(19) Europäisches Patentamt / European Patent Office / Office européen des brevets

(11) **EP 2 269 665 B1**

(12) **EUROPEAN PATENT SPECIFICATION**

(45) Date of publication and mention
of the grant of the patent:
**17.05.2017 Bulletin 2017/20**

(21) Application number: **09738700.5**

(22) Date of filing: **10.04.2009**

(51) Int Cl.:
*A61L 31/00* *(2006.01)*      *A61L 26/00* *(2006.01)*
*A61P 41/00* *(2006.01)*

(86) International application number:
**PCT/JP2009/057359**

(87) International publication number:
**WO 2009/133763 (05.11.2009 Gazette 2009/45)**

(54) **VISIBLE MEDICAL TREATMENT MATERIAL**

SICHTBARES MEDIZINISCHES BEHANDLUNGSMATERIAL

MATÉRIAU DE TRAITEMENT MÉDICAL VISIBLE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **01.05.2008 JP 2008119521**

(43) Date of publication of application:
**05.01.2011 Bulletin 2011/01**

(73) Proprietor: **Terumo Kabushiki Kaisha
Tokyo 151-0072 (JP)**

(72) Inventors:
• **YATABE, Teruyuki
Ashigarakami-gun
Kanagawa 259-0151 (JP)**

• **CHINO, Naotaka
Ashigarakami-gun
Kanagawa 259-0151 (JP)**

(74) Representative: **Dossmann, Gérard et al
Casalonga & Partners
Bayerstrasse 71-73
80335 München (DE)**

(56) References cited:
**WO-A1-97/03114        WO-A1-2004/081055
WO-A1-2005/087289    JP-A- 7 187 998
US-A1- 2003 012 734    US-A1- 2003 224 022
US-A1- 2005 137 272    US-A1- 2006 178 339
US-A1- 2008 058 469**

**Description**

Technical Field

The present invention relates to a visually observable material for medical treatment, which is preferable as an adhesion preventive material, a hemostatic material and the like for biological organisms.

Background Art

In case that invasion occurs in a biological tissue through an operative procedure in a surgery, adhesion is induced via the occurrence of inflammation in the course of tissue curing at a site, so that normal functions of the tissue or organ are blocked by the adhesion, causing sometimes a post-operative complication, disadvantageously. In the region of abdominal cavity, for example, ileus caused post-operatively by adhesion between intestine and peritoneum is a severe complication.

For the purpose of preventing such post-operative adhesion, a biologically absorbable, adhesion preventive material is sometimes used, owing to the expected barrier effect between tissues or organs.

As raw materials for such adhesion preventive materials, so far, there have been proposed for example fibrous cellulose generated by microorganisms, acidic polysaccharides with anionic groups (carboxyl group, sulfonate group) and basic polysaccharides with a cationic group (amino group), including for example dry films of chitosan and polyion complex, inter-polymer complex films of carboxyl-containing polysaccharide and polyether, and aqueous preparations containing polysaccharide dextrin.

It is needless to say that adhesion preventive materials should satisfy clinical needs. Additionally, raw materials therefor should be designed importantly from the standpoint of safety so as to avoid risks such as infections, owing to the use of non-biological materials and to reduce the toxicities of used synthetic materials themselves or decompositions products thereof. Additionally, the materials should be bio-degradable to be absorbed biologically. Furthermore, preferably, adhesion preventive materials can be used immediately on urgent use, involving less preparation procedures to be preliminarily arranged for use during operation and never require any specific apparatus for the use.

As an adhesion preventive material satisfying such needs, the present applicant has proposed an adhesion preventive material comprising a crosslinking polysaccharide derivative with an active ester group covalently binding to active hydrogen-containing group under alkaline conditions, where the active ester group is preliminarily introduced in a polysaccharide known as a highly bio-compatible material (see Patent Reference 1).

Prior Art Literature

Patent Reference

Patent Reference 1: International Publication No. 2005/087289

Other materials are described in US 2003/012734, US 2006/178339 and US 2008/058469.

In particular, US 2003/012734 describes a polymeric coating for a substrate comprising: water, a biocompatible visualization agent that reflects or emits light at a wavelength detectable to a human eye and a biodegradable hydrogel. One application of this coating is prevention of post-operative adhesions.

The biodegradable hydrogel is formed from crosslinked polymers obtained from functional polymers and crosslinkers.

The conditions of the crosslinking reactions depend on the nature of the functional groups. They are preferably conducted in buffered aqueous solutions at pH 5 to 12.

From US 2006/178339, it is known:

- a crosslinkable polysaccharide derivative (A) which has in the side chain at least one active ester group reactive with an hydrogen-containing group capable of forming a crosslinked product through covalent bond upon contact with alkaline water, and
- a composition which comprises the crosslinkable polysaccharide derivative (A) and a pH-adjusting agent (B). As examples of pH-adjusting agent is mentioned sodium hydrogencarbonate, phosphate buffer and acetic acid-ammonia buffer.

US 2008/058469 describes an adhesion preventive material comprising a crosslinking polysaccharide derivative with at least one active ester group in his side chain capable of reacting with an active hydrogen-containing group upon contact with alkaline water. For example, the material comprises a composition which contains said crosslinking polysaccharide derivative and a pH adjuster. As examples of pH-adjusting agents are mentioned a sodium hydrogencarbonate, a phosphoric acid and an acetic acid-ammonia.

Summary of the Invention

Problem that the invention is to Solve

In accordance with the invention, a visually observable material for medical treatment is provided, having great applicability as an adhesion preventive material and the like when applied biologically, which can satisfy demands toward materials for medical treatment, the demands including for example bio-compatibility as described above, no requirement of any specific apparatus, easy preparation on use, sufficient adhesion to biological surface, and an ability to form a crosslinked matrix with great fitness and softness to a subject to be applied, and which has a characteristic feature of forming a visually observable crosslinked matrix, in particular.

Means for Solving the Problems

While the inventor examined crosslinking polysaccharide derivatives reacting with (forming a covalent bond with) active hydrogen-containing group in the presence of an alkali to form a crosslinked matrix as materials for medical treatment as bio-compatible materials, the inventor found that a visually observable crosslinked matrix was formed in case that a carbonate was used as such alkali. Not any bio-adhesive material for medical treatment for clinical use in surgery, which is visually observable, has been known. Further, the visual observability of the actual state (crosslinked gel) of the applied material for medical treatment is extremely useful for the use. In case that the material for medical treatment is used in a spray form, a distinct effect is exerted. Based on the findings, the inventor continued examinations. Using particularly at least one carbonate generating divalent carbonate ion as such carbonate, a visually observable material for medical treatment was practically achieved. It was shown that a mixture with such carbonate could get sufficient usable time, although a mixture of crosslinking polysaccharide derivatives with alkali phosphate salts as prepared on use as a material for medical treatment aggregates to a level not applicable, likely causing insufficiency in usable time. Based on such findings, the following invention is provided.
(1) A material for medical treatment comprising a crosslinking composition comprising at least two components of a crosslinking polysaccharide derivative with an active ester group reactive with active hydrogen in the co-presence of an alkali, where the active ester group is preliminarily introduced into a side chain of a polysaccharide and the crosslinking polysaccharide derivative is not yet crosslinked, and a carbonate including at least one type of carbonate generating divalent carbonate ion in an amount at a mass ratio of 0.03 or more to the polysaccharide derivative.

In accordance with the invention, the term carbonate is used in a wide meaning including carbonates and hydrogen carbonate; and the term "carbonate generating divalent carbonate ion (sometimes referred to as divalent anionic carbonate)" means a carbonate never containing hydrogen. In accordance with the invention, the carbonate generally functions as an alkali.
Further, the crosslinking polysaccharide derivative for use in accordance with the invention even when the crosslinking polysaccharide derivative has no acidic group in the structure of itself is frequently acidic in the form of an aqueous solution. Therefore, the crosslinking polysaccharide derivative exerts self-crosslinking ability in the presence of an alkali. However, the whole crosslinking system is not necessarily under alkaline conditions. Generally, the crosslinking polysaccharide derivative can exert its crosslinking ability even when the crosslinking system is around neutrality or at slight alkalinity exceeding pH 7.
In accordance with the invention, the amount of the divalent anionic carbonate as specified above corresponds to an amount far exceeding the alkali amount minimally needed for the crosslinking of the polysaccharide derivative. Such material for medical treatment in accordance with the invention contains the carbonate at the specified amount to the crosslinking polysaccharide derivative, to form a visually observable gel (crosslinked matrix) encapsulating carbonate gas therein. The divalent anionic carbonate of such carbonate is essential for sufficiently gelatinizing the crosslinking polysaccharide derivative. From the divalent anionic carbonate at the amount or less, a visually observable crosslinked matrix capable of advancing the gelation may be obtained. So as to ensure sufficient usable time for applications as a material for medical treatment, the mass ratio of the divalent anionic carbonate/the polysaccharide derivative is preferably 0.03 or more. The ratio is generally 0.03 to 0.12. For the sole purpose of carbonate gas generation, hydrogen carbonate are advantageously used. However, the single use of the salts cannot progress the gelation sufficiently. In accordance with the invention, therefore, the divalent anionic carbonate given above is essential in the carbonate.
(2) In accordance with the invention, the divalent anionic carbonate is typically sodium carbonate. The divalent anionic carbonate may be sodium carbonate singly or in combination with other carbonates.

(3) Further, the carbonate preferably includes a hydrogen carbonate. In other words, the combination of the divalent anionic carbonate and a hydrogen carbonate is a preferable embodiment of the invention.

A typical example of the hydrogen carbonate is sodium hydrogen carbonate. A typical example of item (3) preferably includes a combination of sodium carbonate and sodium hydrogen carbonate.

In accordance with the invention, the hydrogen carbonate is mainly used as a pH adjuster so as to reduce the strong alkalinity of the divalent anionic carbonate.

In accordance with the invention, the alkali used in combination with the crosslinking polysaccharide derivative is preferably used at pH never affecting human bodies via the application of the material for medical treatment, generally at pH up to 11 at maximum or less, preferably at pH 7 to 10, more preferably at pH 8 to 10.

(4) In a preferable embodiment of the invention, the hydrogen carbonate is contained at a molar ratio of 0.5 to 1 to the divalent anionic carbonate.

(5) The material for medical treatment in accordance with the invention may additionally contain an alkali salt other than the carbonate. Any other alkali salts biologically applicable may be used with no specific limitation, and include salts of phosphoric acid, citric acid, acetic acid and the like. Two types or more of the other alkali salts may be contained therein.

(6) The crosslinking polysaccharide derivative may further contain carboxyl group and/or carboxyalkyl group.

(7) The crosslinking polysaccharide derivative may preferably contain carboxylate group, in an amount of 0.1 to 5 mmol/g, preferably at 0.1 to 2 mmol/g, as the total amount of the active ester group-induced carboxylate group and no such active ester group-induced carboxylate group to the dry mass of the polysaccharide derivative.

Active ester group is an ester group generated by binding an electrophilic group to the carbonyl carbon in the crosslinking polysaccharide derivative of the invention. As the electrophilic group, a group derived from N-hydroxyamine-series compounds is preferable. In that case, the active ester group is succinimide ester group. Such active ester group reacts with active hydrogen-containing group in the presence of an alkali, to form a covalent bond. In the co-presence of an alkali, the crosslinking polysaccharide derivative of the invention induces crosslinking reactions. The pH of the whole reaction system may vary, depending on the applied reaction environment. The pH of the whole reaction system in the applied environment is never specifically limited. The crosslinking polysaccharide derivative when applied biologically binds to an active hydrogen-containing group on the biological surface to exert adhesiveness to the biological surface. Since the polysaccharide backbone contains hydroxyl group corresponding to the active hydrogen-containing group, further, the crosslinking polysaccharide derivative of the invention is self-crosslinkable in the presence of an alkali.

In accordance with the invention, the crosslinking polysaccharide derivative is preferably of a non-salt type. The crosslinking polysaccharide derivative may further contain carboxyl group and/or carboxyalkyl group.

The polysaccharide in which an active ester group is to be introduced (precursor for active esterification) is generally an acid-type polysaccharide with introduced and/or essentially existing carboxyl group and/or carboxyalkyl group. In accordance with the invention, the acid-type polysaccharide is preferably of a non-salt type.

(8) In accordance with the invention, the polysaccharide backbone is an enzymatically degradable polysaccharide, preferably. An embodiment where the polysaccharide is dextrin is preferable. No report about disadvantages of dextrin as a biologically applicable material is issued.

A crosslinking composition comprising the crosslinking polysaccharide derivative and the carbonate can ensure a long usable time, hardly causing aggregation. In case that aggregation occurs, a mixture prepared on use cannot be used. Herein, the term "usable time" means a time period when the gel-forming ability can be retained. It is needless to say that the same effect is exerted from a crosslinking composition comprising a crosslinking dextrin derivative and a carbonate.

(9) The material for medical treatment in accordance with the invention is useful as an adhesion preventive material, a hemostatic material, a clinically adhesive material and the like.

Until use, the crosslinking composition as the material for medical treatment in accordance with the invention generally comprises at least two components of the crosslinking polysaccharide derivative not yet crosslinked and the carbonate, with no specific limitation to the forms of the individual components. As to the forms until use, both or any one of the individual components may satisfactorily be packaged separately in the form of a powder or an aqueous solution. On use, the crosslinking polysaccharide derivative may be applied in the form of a powder. So as to obtain the visually observable effect, the crosslinking polysaccharide derivative is preferably applied in a solution state. Preferably, the crosslinking polysaccharide derivative is used in the form of aerosol or paste.

(10) A particularly preferable mode of using the material for medical treatment in accordance with the invention is a fluid composition for spraying (aerosol). By spraying and applying the material for medical treatment in a fluid form to a needed site, carbonate gas is readily encapsulated in the crosslinked matrix, so that a visually observable crosslinked matrix can effectively be obtained.

The individual components of the crosslinking polysaccharide derivative not yet crosslinked and the carbonate are sprayed on the same applicable site, using a sprayer preferably with two or more nozzles or with a nozzle with a mixing part in the channel.

In accordance with the invention, there is provided a method for forming a visually observable crosslinked matrix by applying the material for medical treatment to a desired site.

(11) The material for medical treatment can form the visually observable matrix encapsulating carbonate gas therein. The resulting visually observable crosslinked matrix is also encompassed within the scope of the invention.

If needed, the material for medical treatment in accordance with the invention may further contain polymers and other components, other than the crosslinking polysaccharide derivative. The other polymers include for example polymers with two or more primary amino groups and/or thiol groups within one molecule. The other polymers include polymers with backbones of polyalkylene glycol, polypeptide and polysaccharide. The other components include preservatives for the polysaccharide derivative, for example trehalose.

These other polymers may be in a form different from those of the individual components. Otherwise, the other polymers may be in mixture forms in powder or solution with the crosslinking polysaccharide derivative.

Advantages of the Invention

The crosslinking polysaccharide derivative in accordance with the invention essentially has a biologically and chemically safe profile because the backbone of the crosslinking polysaccharide derivative is never a material derived from a biological origin and from chemical substances with a potential toxicity. Therefore, the crosslinking polysaccharide derivative is an excellent bio-compatible material. Furthermore, the crosslinking polysaccharide derivative exerts self-crosslinking ability in the co-presence of an alkali, never requiring any specific device, being prepared readily on use, lessening preparative procedures to be preliminarily done for use, being capable of rapidly complying with urgent application and being used with any person in a simple fashion. Additionally, the crosslinking polysaccharide derivative can form a crosslinked matrix with excellent fitness and softness to a person to be applied. The material for medical treatment comprising the crosslinking polysaccharide derivative not yet crosslinked in combination with the carbonate is greatly convenient for biological application, owing to the formation of the visually observable crosslinked matrix.

Brief Description of the Drawing

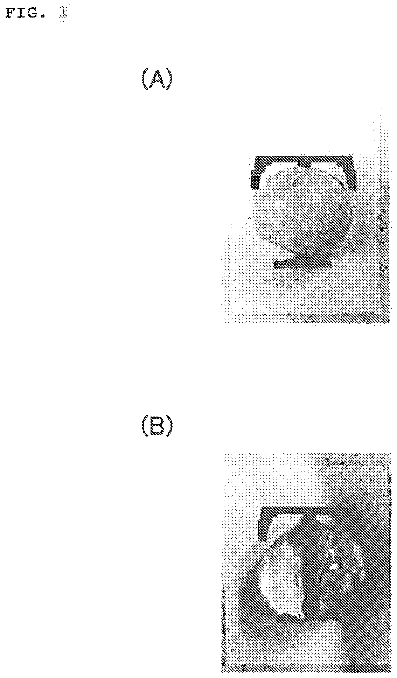

[Fig. 1] A digital picture showing the results of a test for visual observation in Test Example 1.

Mode for Carrying out the Invention

The invention is now specifically described below.

The crosslinking polysaccharide derivative of the invention contains at least one active ester group reactive with active hydrogen in the co-presence of an alkali, where the active ester group is preliminarily introduced in a side chain of a polysaccharide, and therefore induces a crosslinking reaction through the active hydrogen-containing group in the co-presence of an alkali.

The polysaccharide as a raw material into which the active ester group is preliminarily introduced will be described below. Because a polysaccharide molecule essentially has hydroxyl group, namely active hydrogen-containing group in itself, the polysaccharide with an active ester group introduced therein is now provided with both active ester group and active hydrogen-containing group within one molecular chain, so that the polysaccharide molecule exerts self-crosslinking ability under the reaction conditions. The self-crosslinking ability means that the active ester group and the active hydrogen-containing group react together intra- molecularly within one molecule of the polysaccharide derivative or inter-molecularly in the molecules thereof , to form covalent bonds. In case that an active hydrogen-containing group on the biological surface is used for the reaction, additionally, the crosslinking polysaccharide derivative exerts adhesiveness to the biological surface. Herein, the term molecule in the term "one molecular chain" or "intra-molecularly" means a single molecule within a covalently bound continuous zone.

In the specification, the crosslinking polysaccharide derivative is sometimes referred to as actively esterified polysaccharide. Therefore, the crosslinking polysaccharide derivative may sometimes be referred to as polysaccharide derivative in a simple fashion.

The polysaccharide derivative in accordance with the invention is an actively esterified polysaccharide, essentially having a polysaccharide backbone. Hence, the polysaccharide derivative is now described, following the steps of the method for the active esterification of polysaccharide (the method for producing a polysaccharide derivative) hereinbelow.

In accordance with the invention, satisfactorily, the active ester group to be introduced in a polysaccharide may be any active ester group reactive with an active hydrogen-containing group to form a covalent bond in the co-presence of an alkali and in the presence of water. Generally, such active ester group is contained in polysaccharide molecules themselves or is a group generated by binding an electrophilic group to the carbonyl carbon in the carboxyl group and/or methylcarboxyl group introduced therein, and the group is stronger than those of common esters.

When the active ester group is specifically represented as "-COOX", the electrophilic group forming "-OX" as the alcohol moiety may preferably be a group introduced from N-hydroxyamine-series compounds. Because N-hydroxyamine-series compounds are relatively inexpensive raw materials, industrial introduction of the active ester group can be done readily. Specifically, the N-hydroxyamine-series compounds for forming "-OX" typically include for example N-hydroxysuccinimide, N-hydroxynorbornene-2,3-dicarboximide, 2-hydroxyimino-2-cyanoacetate ethyl ester, 2-hydroxyimino-2-cyanoacetamide, and N-hydroxypiperidine.

In accordance with the invention, a single type or two types or more of such active ester group may exist in the polysaccharide derivative.

Among the active ester groups, succinimide ester group is preferable.

In accordance with the invention, the polysaccharide with the active ester group introduced therein to compose the main backbone of the polysaccharide derivative may contain two units or more of monosaccharide structures in the main backbone, with no specific limitation. Such polysaccharide includes polysaccharides formed via covalent bonding of monosaccharides such as arabinose, ribose, xylose, glucose, mannose, galactose, fructose, sorbose, rhamnose, fucose and ribodesose, disaccharides such as trehalose, sucrose, maltose, cellobiose, gentiobiose, lactose, and melibiose, and polysaccharides of three or more monosaccharide units such as trisaccharides including raffinose, gentianose, melezitose, and stachyose, or the polysaccharides with additional functional groups introduced therein. In accordance with the invention, such polysaccharides may be from natural origins or may be artificially prepared synthetically. Further, the polysaccharide derivative of the invention may comprise a single polysaccharide backbone or two types or more of polysaccharide backbones.

Any mass average molecular weight of the polysaccharide as the main backbone of the polysaccharide derivative may be satisfactory with no specific limitation. The polysaccharide of a mass average molecular weight of 5,000 to 2,500,000 is preferred, which corresponds to several tens to several thousands of the monosaccharides, the disaccharides and the polysaccharides of three or more monosaccharide units, after binding. The reason is that such polysaccharide can readily adjust gel hardness after the polysaccharide derivative of the invention crosslinks together and that plural active ester groups and active hydrogen-containing groups can readily be introduced into one molecular chain of the polysaccharide derivative. Preferably, the polysaccharide is a polysaccharide of Mw (mass average molecular weight) of 10,000 to 1,000,000.

For the introduction of the active ester group "-COOX" into the polysaccharide, generally, a polysaccharide with carboxyl group and/or carboxyalkyl group (these are sometimes referred generically to as carboxylate group) (sometimes referred to as acid-type polysaccharide) is used. The carboxyalkyl group herein means a functional group of carboxyl group bound to the alkyl backbone, as illustrated by carboxymethyl group, carboxyethyl group, carboxypropyl group, carboxyisopropyl group and carboxybutyl group.

The acid-type polysaccharide may be of any acid type at the stage of a precursor for the crosslinking polysaccharide derivative, which may be a natural polysaccharide essentially containing carboxylate group therein or may be a polysaccharide without any essential carboxylate group but with carboxyl group and/or carboxyalkyl group introduced therein. Among such acid-type polysaccharides, natural polysaccharides with carboxyl group, carboxylated polysaccharides with carboxyl group introduced therein, carboxymethylated polysaccharides with carboxymethyl group introduced therein, and carboxylethylated polysaccharides with carboxyethyl group introduced therein are preferable. Natural polysaccharides with carboxyl group, carboxylated polysaccharides with carboxyl group introduced therein, and carboxymethylated polysaccharides with carboxymethyl group introduced therein are more preferable.

The natural polysaccharides with carboxylate group existing essentially therein include for example but are not limited to pectin containing galacturonic acid, and hyaluronic acid. For example, pectin includes "GENUE pectin" of CP Kelco (Denmark) while hyaluronic acid includes "Hyaluronic acid FCH" of Kibun Co., Ltd. As such, generally, commercially available polysaccharides may be used. Pectin is a polysaccharide containing galacturonic acid as the main component. About 75 to 80 % or more of pectin comprises galacturonic acid. Other components in pectin mainly comprise other saccharides. Pectin is a polysaccharide comprising galacturonic acid and other saccharides bound together at the ratio described above. Hyaluronic acid is used as an auxiliary agent in ophthalmologic surgery and a therapeutic agent of knee osteoarthritis. Hyaluronic acid never contains galacturonic acid.

In accordance with the invention, preferably, the carboxyl group and/or carboxyalkyl group in the polysaccharide derivative is preferably of the "non-salt type" with no coordinate salt. The final polysaccharide derivative is preferably not in a salt form. Herein, the term "salt" encompasses inorganic salts of alkali metals and alkali earth metals, and halogen salts of quaternary amines such as tetrabutylammonium (TBA) and chloromethylpyridinium iodide. The term "non-salt type" means no coordination with these "salts". The phrase "not in a salt form" means no content of these salts.

The polysaccharides having the carboxyl group and/or carboxyalkyl groups introduced therein include but are not limited to dextran, pullulan and dextrin.

The dextrin is used as substitute plasma. As the dextran, "Dextran T fractions" of Amersham Bio-sciences Co., Ltd. (Japan) is used; as the pullulan, "Pullulan PI-20" of Hayashibara Co., Ltd. (Japan) is used. Pullulan is also used as an additive for medicines including oral pharmaceutical agents, preferably with less biological contaminants such as endotoxin.

Dextrin is a hydrolysis product of carbohydrate and is a mixture of glucose polymers with different molecular chain lengths. The glucose unit in dextrin is bound together mainly via $\alpha$-1,4-bond, including a certain ratio of $\alpha$-1,6-bond. In accordance with the invention, the starch type as a raw material of dextrin is not specifically limited. Therefore, the ratio of existing $\alpha$-1,6-bond is not specifically limited.

Taking account of ready availability, physico-chemical properties on use, ready handleability, and film-forming ability, the dextrin to be used in accordance with the invention typically has Mw of about 10,000 to 100,000.

Any generally commercially available polysaccharides may be used in accordance with the invention. The polysaccharide with a successful outcome in the medical applications descried above is a polysaccharide preferably used owing to the safety profile in accordance with the invention. Among those described above, not any anaphylaxis shock has been reported about dextrin, which has been used successfully in peritoneum dialysis. Thus, dextrin is a particularly preferable polysaccharide, in view of no disadvantages reported in biological applications.

Using known oxidation reactions, the carboxylation of the polysaccharides can be done with no specific limitation. Any type of the carboxylation may be satisfactory and includes for example oxidation with dinitrogen tetraoxide, oxidation with fuming sulfuric acid, oxidation with phosphoric acid, oxidation with nitric acid and oxidation with hydrogen peroxide. For the individual oxidations, generally known reactions using such reagents may be selected. The individual reaction conditions may appropriately be determined, depending on the amount of carboxyl group to be introduced. By suspending a raw material polysaccharide in chloroform or carbon tetrachloride, and adding dinitrogen tetraoxide to the resulting suspension, for example, the hydroxyl group of the polysaccharide is oxidized to prepare a carboxylated polysaccharide (carboxylated product of polysaccharide).

Additionally, known carboxyalkylation reactions for polysaccharides can be used as the carboxyalkylation reaction, with no specific limitation. In case of the carboxymethylation of a polysaccharide, specifically, a reaction may be selected, comprising preparing the polysaccharide to an alkalinity and using monochloroacetic acid. The reaction conditions can appropriately be determined, depending on the amount of carboxymethyl group to be introduced.

As a method for introducing carboxylate group into a polysaccharide in accordance with the invention, any method for the carboxylation or the carboxyalkylation may be used, with no specific limitation. From the standpoints that the molecular weight of the polysaccharide is less reduced via the introduction reaction of carboxyl group and that the amount of carboxyl group to be introduced can relatively readily be controlled, the carboxyalkylation, particularly carboxymethylation is preferable.

In accordance with the invention, further, the introduction of carboxylate group is not specifically limited to polysaccharides having themselves no carboxylate group therein. Carboxyl group and/or carboxymethyl group may additionally be introduced in natural polysaccharides having carboxylate group themselves, for example hyaluronic acid. In modifying the carboxyl group and/or carboxymethyl group of an acid-type polysaccharide to active ester, as described above, the acid-type polysaccharide may be used singly or a combination of two types or more of acid-type polysaccharides may be used.

The amount of carboxylate group per 1 g of the dry mass of the acid-type polysaccharide to be used for active esterification (provided that the group is assumed as one molecule) is generally 0.1 to 5 mmol/g, preferably 0.4 to 3 mmol/g, more preferably 0.6 to 2 mmol/g. When the ratio of the carboxylate group is less than 0.1 mmol/g, the number of active ester groups functioning as crosslinking points as induced in the group may frequently be insufficient. When the ratio of the carboxylate group is more than 5 mmol/g, the resulting polysaccharide derivative (not yet crosslinked) is slightly dissolved in a solvent including water.

In accordance with the invention, the final crosslinking polysaccharide derivative may satisfactorily contain carboxylate group (carboxyl group and/or carboxyalkyl group) not yet actively esterified, together with active ester. In this case, the total of active ester group and carboxylate group in the final crosslinking polysaccharide derivative is the amount of the carboxylate group, as described above.

The active esterification method of the acid-type polysaccharide (the method for producing the polysaccharide derivative) includes for example but is not specifically limited to a method comprising allowing the acid-type polysaccharide to react with an agent for introducing an electrophilic group in the presence of a dehydration and condensation agent, and a method of an ester interchange reaction comprising introducing an active ester group from a compound with an active ester group into a polysaccharide. Among them, the former method is preferable for the invention. Accordingly, the method (sometimes referred to as the inventive method) is mainly described hereinbelow.

In carrying out the preferable method in accordance with the invention, generally, the acid-type polysaccharide is prepared as a solution of a non-protonic polar solvent for the reaction. More specifically, the method comprises a solution preparative step of dissolving a polysaccharide with carboxyl group or carboxyalkyl group in a non-protonic polar solvent, and a reaction step of adding an agent for introducing an electrophilic group and a dehydration and condensation agent to the resulting solution to prepare the carboxyl group or carboxyalkyl group of the polysaccharide as active ester, and additionally comprises a purification step of the resulting reaction product and a drying step.

At the solution preparative step, a polysaccharide is added to the solvent for heating to 60°C to 120°C, to dissolve the polysaccharide in the non-protonic polar solvent.

Among the illustrated polysaccharides, a polysaccharide dissolvable in a non-protonic polar solvent at a temperature of 60°C to 120°C is preferably used as an acid-type polysaccharide to be actively esterified by the method. Specifically, the polysaccharide to be used in the reaction for introducing an electrophilic group is preferably of an acid type where the counter cation species of the carboxyl group or carboxymethyl group is proton, in view of the solubility in the non-protonic polar solvent. The acid type has the same meaning as that of the "non-salt type" since the acid type is not in a salt form.

The term "non-protonic polar solvent" is a polar solvent with no proton capable of forming a hydrogen bond with a nucleophilic agent with an electrically positive functional group. The non-protonic polar solvent for use in the inventive method includes but is not limited to dimethyl sulfoxide (DMSO), N,N-dimethylformamide, N-methyl-2-pyrrolidone, N,N-dimethylacetamide, and 1,3-dimethyl-2-imidazolidinone. In view of the great solubility of the polysaccharide in a solvent, dimethyl sulfoxide is preferably used.

At the reaction step, an agent for introducing an electrophilic group and a dehydration and condensation agent are added to such a solution of the acid-type polysaccharide as described above, to modify the carboxyl group and/or carboxymethyl group of the polysaccharide as active ester. The reaction temperature for the active esterification includes but is not limited to preferably 0°C to 70°C, more preferably 20°C to 40°C. The reaction time varies depending on the reaction temperature, but is generally one to 48 hours, preferably 12 hours to 24 hours.

The term "agent for introducing an electrophilic group" is a reagent for introducing an electrophilic group in carboxyl group or carboxyalkyl group to modify the group to active ester group. As the agent for introducing an electrophilic group, active ester-inducing compounds for common use in peptide synthesis can be used with no specific limitation. One of the examples is an N-hydroxyamine-series active ester-inducing compound. The N-hydroxyamine-series active ester-inducing compound includes for example but is not limited to N-hydroxysuccinimide, N-hydroxynorbornene-2,3-dicarboximide, 2-hydroxyimino-2-cyanoacetate ethyl ester, 2-hydroxyimino-2-cyanoacetamide, and N-hydroxypiperidine. Among them, N-hydroxysuccinimide is preferable owing to the successful outcome in the field of peptide synthesis and the ready commercial availability.

In modifying carboxyl group or carboxyalkyl group via the introduction of the agent for introducing an electrophilic group to active ester groups, the "dehydration and condensation agent" works for drawing out one water molecule generated from the condensation between carboxyl group or carboxyalkyl group and an agent for introducing an electrophilic group, ie. dehydrating the water molecule and then binding both the resulting group and the resulting agent together via ester bond. The dehydration and condensation agent includes for example but is not limited to 1-ethyl-3-dimethylaminopropylcarbodiimide hydrochloride salt (EDC·HCL), and 1-cyclohexyl-(2-morphonyl-4-ethyl)-carbodiimide·meso p-toluenesulfonate. Among them, 1-ethyl-3-dimethylaminopropylcarbodiimide hydrochloride salt (EDC·HCL) is preferable owing to the successful outcome in the field of peptide synthesis and the ready commercial availability.

At the purification step, the agent for introducing an electrophilic group and the dehydration and condensation agent, which are left unreactive, are removed along with reaction byproducts from the reaction solution by general measures such as reprecipitation, filtration and/or washing, after completion of the reaction step, to obtain the polysaccharide derivative of the invention.

At the drying step, the resulting polysaccharide derivative is dried by a method for general use so as to remove the washing solvent from the polysaccharide derivative obtained at the purification step.

So as to obtain the polysaccharide derivative in a powdery form, the dried polysaccharide derivative is disintegrated or pulverized, and the range of the particle sizes therein is arranged by adjusting the particle sizes if necessary. So as to reduce the particle size, the polysaccharide derivative is frozen and pulverized, and is ground with a mill and/or graded, with no specific limitation. After disintegration or pulverization, the resulting product may be sifted to adjust its particle size distribution appropriately. With no specific limitation, the mean particle size is preferably a particle size of several tens nm to several hundreds $\mu$m.

In accordance with the invention, as described above, the final amount of active ester group in the polysaccharide derivative is preferably 0.1 to 2 mmol/g. So as to obtain such polysaccharide derivative as described above, the amount of active ester group to be introduced into the carboxyl group of a raw material polysaccharide for active esterification can be controlled.

So as to control the amount of active ester group to be introduced therein, the amounts of an agent for introducing an electrophilic group and a dehydration and condensation agent in mixture may be adjusted. Specifically, the conditions for the addition preferably satisfy that the ratio (Z/X) of the molar number (Z mmol) of a dehydration and condensation agent to the molar number (X mmol) of total carboxyl groups in a polysaccharide is 0.1 < Z/X < 50 at the reaction temperature. When Z/X is smaller than 0.1, the amount of the dehydration and condensation agent added is so less that the reaction efficiency is low, leading to difficulty in achieving a desired introduction ratio of active ester group. When Z/X is larger than 50, the amount of the dehydration and condensation agent added is so large that the resulting polysaccharide derivative is hardly dissolvable in water although the introduction ratio of active ester group is large.

The molar number (Y mmol) of an agent for introducing an electrophilic group to the molar number (X mmol) of total carboxyl groups in a polysaccharide is not specifically limited as long as the agent for introducing an electrophilic group is added at a reaction amount or more, depending on the introduction ratio of active ester group. Preferably, the conditions for the addition are such that the ratio satisfies 0.1 < Y/X < 100, with no specific limitation.

After the introduction of active ester group, the polysaccharide derivative of the invention generally contains hydroxyl group of the glucopyranose ring in the backbone molecule of the polysaccharide and has therefore active hydrogen-containing group in itself. The active hydrogen-containing group within the molecule is not limited to the active hydrogen-containing group described above but contains another active hydrogen-containing group introduced within the molecule, if necessary. In this case, a single type or two types or more of active hydrogen-containing groups may be contained in the polysaccharide derivative.

The polysaccharide derivative of the invention may widely contain functional groups such as known elements and atomic groups in addition to the active ester group and active hydrogen-containing group within a range never affecting the characteristic aspects of the invention.

Such functional groups specifically include halogen elements such as fluorine, chloride, bromine and iodine; carboxyl group; carboxyalkyl groups such as carboxymethyl group, carboxyethyl group, carboxypropyl group and carboxyisopropyl group; and silyl group, alkylenesilyl group, alkoxysilyl group and phosphate group. Such functional group may be introduced singly or in combination of two types or more thereof.

The polysaccharide derivative for use in accordance with the invention has at least one active ester group described above within the molecule. So as to form a crosslinked matrix, generally, the polysaccharide derivative has two or more active ester groups within one molecule. Because a polysaccharide derived from a raw material from natural origins is frequently a mixture of molecules with different molecular weights, the ratio of the content of active ester group is appropriately represented by the amount of active ester group per one gram of the dry mass thereof. Depending on the purpose of the use of the polysaccharide derivative, the ratio of the content of active ester group varies. Generally, the ratio of the content of active ester group is preferably 0.1 to 5 mmol/g.

The ratio (%) of active ester group to be introduced can be represented by the ratio (AE/TC) of the molar content (AE) of active ester group in the resulting polysaccharide derivative to total carboxyl groups (TC) contained in an acid-type polysaccharide, after multiplication with 100.

The ratio of active ester group to be introduced can be determined by a method described in for example Biochemistry Vol. 14, No. 7 (1975), p. 1535 - 1541.

In an example that the active ester group is an ester group introduced by N-hydroxysuccinimide (sometimes abbreviated as NHS hereinafter), specifically, the NHS introduction ratio can be determined on the basis of the ratio of the content of active ester group in the resulting polysaccharide derivative to the content of carboxyl group and the content of carboxymethyl group existing in each unit mass of the acid-type polysaccharide in the following way.

So as to prepare a standard curve of N-hydroxysuccinimide (NHS), aqueous NHS standard solutions at 0.1, 0.2, 0.5, 1.0, 2.5, 5.0 and 10 mM are prepared. 0.2 mL of aqueous 2N sodium hydroxide solution is added to 1 mL of each aqueous NHS standard solution, for heating at 60°C and agitation for 10 minutes. After cooling, 1.5 mL of 0.85N hydrochloric acid and 0.75 mL of 0.05% $FeCl_3$/1N hydrochloric acid solution are added, to determine the absorbance at an absorption wave length of 500 nm, using a spectrophotometer ($FeCl_3$ manufactured by Wako Pure Chemical Co., Ltd.). The concentration of each aqueous NHS solution is plotted on X axis, while the absorbance is plotted on Y axis, for linear approximation, to obtain the following numerical equation formula (2) for calculating the NHS concentration.

$$Y = \alpha X + \beta \qquad \cdots\cdots \quad (2)$$

X = NHS concentration (mM)
Y = absorbance at a wave length of 500 nm
$\alpha$ = 0.102 (slope)
$\beta$ = 0.0138 (section)

R = 0.991 (coefficient of correlation)

By multiplying X (mM) calculated on the basis of absorbance with the volume of an assay solution (3.45 mL), the NHS content (C mmol) in a sample described below can be determined.

Then, 0.01 g of the actively esterified polysaccharide is weighed and added to 1 mL of pure water, for agitation at 25°C for 3 hours, to which 0.2 mL of aqueous 2N sodium hydroxide solution is added, for heating at 60°C and agitation for 10 minutes. After cooling to ambient temperature, 1.5mL of 0.85N hydrochloric acid is added. From the resulting solution containing insoluble matters, the insoluble matters are removed using a filter cotton. Subsequently, 0.75 mL of 0.05% $FeCl_3$/1N hydrochloric acid is added to assay the absorbance at a wave length of 500 nm. When the absorbance measured is above the absorbance at the 5 mM concentration of the NHS standard solution, the sample is diluted with pure water (H as dilution magnification). Using the numerical equation formula (2) for calculating the NHS concentration, based on the assayed absorbance, the content (C mmol) of NHS group in an actively esterified polysaccharide is calculated. Subsequently, the NHS introduction ratio in the actively esterified polysaccharide is determined according to the following numerical equation formula (3).

$$\text{NHS introduction ratio (\%)} = [(C \times H)/0.01]/B \times 100 \cdots$$

$$(3)$$

B: amount (mmol/g) of total carboxyl groups in the acid-type polysaccharide in actively esterified polysaccharide
C: content (mmol) of NHS group in actively esterified polysaccharide

Provided that the introduction ratio of active ester group is less than 100 % above, the resulting crosslinking polysaccharide derivative has carboxyl group and/pr carboxymethyl group of the acid-type polysaccharide, together with active ester group.

A preferable embodiment of the crosslinking polysaccharide derivative of the invention is NHS dextrin, where dextrin of Mw 70, 000 to 90, 000 is the polysaccharide and the introduction amount of NHS group is 0.5 to 0.7 mmol/g and the introduction amount of carboxymethyl (CM) group is 0.7 to 0.9 mmol/g.

Such polysaccharide derivative as described above can react with an active hydrogen-containing group in the co-presence of an alkali, to form a crosslinked matrix. When a property capable of forming a crosslinked structure via covalent bond within the intra-molecular chains or inter-molecular chains per se with no use of any crosslinking agent is defined as self-crosslinking property, the polysaccharide derivative of the invention is a polysaccharide with the self-crosslinking property. The co-presence of an alkali means a condition involving the presence of water above pH 7. For the use as the material for medical treatment in accordance with the invention, the polysaccharide derivative can form a crosslinked matrix in the co-presence of an alkali at a temperature for the crosslinking reaction, which is generally a biological temperature, because heat contributes substantially less to the crosslinking reaction. The material for medical treatment in accordance with the invention contains a divalent anionic carbonate functioning as an alkali, in an amount corresponding to a mass ratio of 0.03 or more, generally 0.03 to 0.12 to the polysaccharide derivative.

As the divalent anionic carbonate, any divalent anionic carbonate generating a divalent carbonate ion (2-) in water may be satisfactory and includes for example but is not specifically limited to sodium carbonate, potassium carbonate, calcium carbonate and magnesium carbonate.

The divalent anionic carbonate is generally at least sodium carbonate singly or in combination with other divalent anionic carbonates.

In accordance with the invention, hydrogen carbonates usable in combination with the divalent anionic carbonate include for example sodium hydrogen carbonate and potassium hydrogen carbonate. In the combination of the divalent anionic carbonate and the hydrogen carbonates, the molar ratio of the hydrogen carbonates/the divalent anionic carbonate is preferably 0.5 to 1.

A preferable combination example of the divalent anionic carbonate and a hydrogen carbonate is a combination of sodium carbonate and sodium hydrogen carbonate.

Alkali salts to be contained in the material for medical treatment in accordance with the invention other than the carbonate described above include for example phosphate buffers such as disodium hydrogen phosphate and potassium dihydrogen phosphate, citrate buffers such as trisodium citrate, and acetate-ammonia buffers.

In accordance with the invention, "the crosslinked structure" means that a covalent bond is formed in intra-molecular chains of one molecule of the polysaccharide derivative of the invention and/or in inter-molecular chains of plural such molecules, leading to a three-dimensional network structure of the molecular chains of the polysaccharide derivative. Via crosslinking, the active ester group and the active hydrogen-containing group may bind together within one molecular chains and may also bind together within plural such molecules via covalent bonds, for crosslinking. The polysaccharide derivative of the invention, which is water-soluble before the crosslinking reaction, forms a crosslinked structure following the progress of the reaction, so that the fluidity is reduced, leading to the formation of a water-insoluble mass (hydrous gel) to form a crosslinked polysaccharide (crosslinked matrix). The crosslinked matrix of the invention encapsulates carbonate gas, so that the existence of the gel can be verified visually.

In accordance with the invention, the active hydrogen-containing group responsible for the reaction with active ester group may be any active hydrogen-containing group existing on the biological surface and having reactivity with the active ester group to form a covalent bond under the specific reaction condition of the invention, where the type of the active hydrogen-containing group is not specifically limited. The active hydrogen-containing group includes for example hydroxyl group, primary or secondary amino groups and thiol groups.

If needed, the material for medical treatment in accordance with the invention may further contain polymers other than the polysaccharide derivative. Other polymers include for example polymers with two or more primary amino groups and/or thiol groups within one molecule. Other polymers include for example polymers with backbones of polyalkylene glycol, polypeptide and polysaccharides.

Other polymers include preferably but are not limited to polymers with two or more primary amino groups, thiol groups or hydroxyl groups within one molecule. Specifically, the other polymers include for example polyalkylene glycol derivatives, polypeptide, polysaccharides and derivatives thereof. Two types or more of the other polymers may be used in combination.

The polyalkylene glycol derivatives include polyethylene glycol (PEG) derivatives, polypropylene glycol derivatives, polybutylene glycol derivatives, and block copolymer derivatives and random copolymer derivatives of polypropylene glycol-polyethylene glycol. The fundamental polymer backbone of polyethylene glycol derivatives includes for example ethylene glycol, diglycerol, pentaerythritol and hexaglycerol. Polypeptide includes for example but is not limited to collagen, gelatin, albumin or polylysine. The polysaccharide includes for example but is not limited to pectin, hyaluronic acid, chitin, chitosan, carboxymethyl chitin, carboxymethyl chitosan, chondroitin sulfate, keratin sulfuric acid, keratosulfuric acid, heparin or derivatives thereof.

The material for medical treatment in accordance with the invention can contain widely known additives within a range never affecting the characteristic aspects of the invention. In that case, particularly, additives biologically acceptable may preferably be used. The additives include for example but are not limited to settling catalysts, fillers, plasticizers, softening agents, stabilizers, dehydration agents, colorants, dripping-preventing agents, thickeners, adjusting agents of physico-chemical properties, reinforcing agents, precipitation-preventing agents, anti-aging agents, retardants, antioxidants, ultraviolet absorbents, pigments, solvents, carriers, excipients, preservatives, binders, antioxidants, expanding agents, isotonic agents, auxiliary dissolution agents, preservatives, buffers and diluents. These may be contained singly or in combination of two types or more thereof.

The additives specifically include water, physiological saline, pharmaceutically acceptable organic solvents, gelatin, collagen, trehalose, polyvinyl alcohol, polyvinyl pyrrolidone, carboxyvinyl polymer, sodium carboxymethyl cellulose, sodium polyacrylate, sodium alginate, water-soluble dextran, sodium carboxymethyl starch, pectin, methyl cellulose, ethyl cellulose, gum xanthane, gum Arabic, tragacanth, casein, agar, diglycerin, propylene glycol, polyethylene glycol, Vaseline, paraffin, stearyl alcohol, stearic acid, human serum albumin (HSA), mannitol, sorbitol, lactose, PBS, non-ionic surfactants, bio-degradable polymers, serum-free culture media, surfactants acceptable as pharmaceutical additives or biologically acceptable buffers at physiological pH.

Depending on the site to be used, carriers are selected, appropriately from those described above or as a combination thereof. However, the carriers are never limited to them.

Specifically, trehalose is preferably used as a preservative of the polysaccharide derivative.

In the material for medical treatment in accordance with the invention, generally, the polysaccharide derivative and the carbonate are not in contact with each other until mixing for use. In case that the material for medical treatment contains other components, further, the other components are in a state of contact with the polysaccharide derivative or are provided at separated states, until use, although the state or the states depend on their types.

A preferable embodiment of the material for medical treatment in accordance with the invention can be prepared as a preparation such as aerosol or paste, with an appropriate propellant. A particularly preferable embodiment thereof is a fluid composition for spraying (aerosol). By spraying and applying the material for medical treatment in the fluid state onto a needed site, carbonate gas is readily encapsulated in the crosslinked matrix, so that a visually observable crosslinked matrix can effectively be obtained.

The individual components of the crosslinking polysaccharide derivative not yet crosslinked and the carbonate are sprayed on the same site of application, preferably using a sprayer with two or more nozzles or a sprayer with a nozzle with a mixing part in the channel.

The material for medical treatment in accordance with the invention is useful as an adhesion preventive material, a hemostatic material, a clinical adhesion material and the like. The material for medical treatment is particularly useful as an adhesion preventive material.

The adhesion preventive material means a substance to be used for the purpose of preventing adhesion at or around a biological site for the prevention of adhesion, which comprises a safe material with a low toxicity for biological organisms and which is biologically acceptable. The adhesion preventive material may or may not be bio-degradable, preferably bio-degradable. By applying the adhesion preventive material to an intended site to cover the site with a possible occurrence of adhesion, adhesion can be inhibited. Hence, the adhesion preventive material can exert the adhesion preventive effect.

The invention is now described below more specifically. However, the examples are just illustrative. The invention is never limited by them.

Examples

(Synthetic Example 1)

Preparation of NHS dextrin

1. Preparation of a raw material for active esterification Preparation of acid-type polysaccharide carboxymethyl dextrin

(CM dextrin hereinafter)

10 g of dextrin (Sandeck SD#100 as a trade name manufactured by Sanwa Starch and of a mass average molecular weight of 15,000) was dissolved in 62.5 g of pure water in a 500-mL flask. 62.5 g of aqueous 36 wt% sodium hydroxide solution was added for agitation at ambient temperature for 90 minutes. Aqueous chloroacetic acid solution prepared by adding distilled water to 10.91 g (109.1 mmol) of chloroacetic acid to a final weight of 75 g was added to the flask, for reaction at 60°C for 6 hours. After cooling the flask to ambient temperature, 80 mL of aqueous 20% HCl solution was added to obtain a reaction solution containing CM dextrin.

The total volume of the reaction solution was added to a 5-L beaker containing 4450 mL of ethanol and 180 mL of water, under agitation. The deposit was collected by filtration, washed first with 2L of aqueous 90 % ethanol solution and 2L of ethanol, for drying under reduced pressure at ambient temperature for 24 hours, to obtain CM dextrin. The amount (mmol/g) of carboxyl group and carboxymethyl group in the CM dextrin obtained above was determined, which was 0.8 mmol/g. The method for determining the amount of the groups is described below.

Determination of carboxyl group and carboxymethyl group: 0.2 g of acid-type polysaccharide was weighed (A (g)) and added to a mixture solution of 20 mL of aqueous 0.1 mol/L sodium hydroxide solution and 10 mL of aqueous 80 vol% methanol solution, for agitation at 25°C for 3 hours. Three drops of 1.0 W/V % phenolphthalein/aqueous 90 vol% ethanol solution as an indicator was added to the resulting solution, for an acid-base reverse titration using 0.05 mol/L sulfuric acid, to assay the volume ($V_1$ mL) of 0.05 mol/L sulfuric acid used (phenolphthalein manufactured by Wako Pure Chemical Co. , Ltd.). Additionally, the volume ($V_0$ mL) of 0.05 mol/L sulfuric acid used was measured for a blank in the same manner except for no addition of the acid-type polysaccharide. According to the following formula (1), the amount (B mmol/g) of carboxyl group and carboxymethyl group in the acid-type polysaccharide is calculated. The titers of the aqueous 0.1 mol/L sodium hydroxide solution and the 0.05 mol/L sulfuric acid are both 1.00.

$$B = (V_0 - V_1) \times 0.1 \div A \qquad \cdots (1)$$

A: mass (g) of the acid-type polysaccharide
B: amount (mmol/g) of carboxyl group and carboxymethyl group

2. Active esterification of CM dextrin

10 g (8 mmol) of CM dextrin (at the amount of carboxymethyl group =0.8 mmol/g) obtained above and 300 g of DMSO were charged in a 1-L flask, for complete dissolution under agitation at ambient temperature. Subsequently, 9.3 g (80 mmol) of N-hydroxysuccinimide (NHS) (manufactured by Wako Pure Chemical Co., Ltd.) was added for complete dissolution under agitation at ambient temperature. Then, 15.4 g (80 mmol) of 1-ethyl-3-dimethylaminopropylcarbodiimide hydrochloride salt (EDC) (manufactured by Wako Pure Chemical Co., Ltd.) was added, for complete dissolution at ambient temperature under agitation. Subsequently, the resulting solution was agitated at ambient temperature for another 16 hours, to obtain a reaction solution containing NHS dextrin.

Then, the total volume of the reaction solution containing NHS dextrin as obtained above was added to a 3-L beaker containing 2 L of ethanol/acetone = 1:1 (v/v) under agitation. The deposit was filtered (Kiriyama Rohto), and washed first with 800 mL of ethanol/acetone = 1:1 (v/v) and then with 800 mL of acetone on the funnel. The filtered deposit was dried under reduced pressure at ambient temperature for 24 hours, to obtain NHS dextrin.

The NHS introduction ratio in the NHS dextrin obtained above was determined by the method described above, which was 75 %.

(Test Example 1)

Visual Observability Test

0.4 ml of a 20 wt % solution of the NHS dextrin obtained above was measured in a polypropylene test tube, to which 0.1 mL of a mixture solution (pH 9.2) of 0.5M sodium carbonate/0.5M sodium hydrogen carbonate was added in an example or to which 0.1 mL of 1.0M disodium hydrogen phosphate (pH 8.4) was added in a comparative example. The resulting mixtures were left to stand alone for 2 minutes. The reaction products were placed on a sheet with a character printed thereon. The digital pictures are shown in Fig. 1.

As shown in the pictures, the reaction product in the example of the invention (see Fig. 1(A)) is in suspension so no character below can be observed through the product, making the gelation visually observable. The reaction product in the comparative example (Fig. 1(B)) is transparent after gelation, through which the character below can be observed visually.

In the same manner as described above, 0.1 ml of an alkaline agent shown in Table 1 was added. When the character could not be confirmed through a reaction product, it was determined that the reaction product had visual observability (O); and when the character could be confirmed through a reaction product, the reaction product was determined to have no visual observability (X). The results are shown in Table 1.

(Test Example 2)

Assessment of usable time

NHS dextrin was dissolved in RO water (reverse-osmosis filtered water) and left to stand alone for an appropriate time (0 minute, 20 minutes, 30 minutes and 60 minutes), to which a carbonate and/or another alkaline salt shown in Table 1 was added at an amount shown in Table 1 for gentle shaking. 20 seconds after the addition of the individual salts, the test tube was inversed. When the content was not fluid, the resulting test tube was marked as O; when the content was fluid, the resulting test tube was marked as X. The results are shown in Table 1.

As shown in Table 1, NHS dextrin exerted a longer usable time, depending on the combination with a divalent anionic carbonate at an amount specified in accordance with the invention, and the NHS dextrin additionally formed a visually observable crosslinked gel (Test Nos. 1, 2, 4 and 5). On the contrary, when no divalent anionic carbonate was used, crosslinked gels of NHS dextrin with phosphate-series and citrate-series alkalis never exerted any visual observability (Test Nos. 9, 10). Additionally, these had insufficient usable time.

When a divalent anionic carbonate was used at an amount below the amount in accordance with the invention, no visual observability was obtained, involving insufficient usable time (Test No.3); otherwise, the resulting material for medical treatment had insufficient usable time even when the material for medical treatment had visual observability (Test No.6).

[Table 1]

Table 1

| Alkaline material | Test No. | 1 | 2 | 3 | 4 | 5 | 6 | 7 | 8 | 9 | 10 |
|---|---|---|---|---|---|---|---|---|---|---|---|
| Sodium carbonate | Concentration (mol/L) | 1 | 0.5 | 0.2 | 0.5 | 0.33 | 0.25 | 0.5 | 0.5 | - | - |
| Sodium hydrogen carbonate | | - | | | 0.5 | 0.67 | 0.75 | - | - | - | - |
| Disodium hydrogen phosphate | | - | | | - | | | 0.5 | - | 1.0 | |
| Disodium citrate | | - | | | - | | | - | 0.5 | - | 1.0 |

(continued)

| Alkaline material | Test No. | 1 | 2 | 3 | 4 | 5 | 6 | 7 | 8 | 9 | 10 |
|---|---|---|---|---|---|---|---|---|---|---|---|
| pH of alkali | | 11.3 | 11.2 | 10.4 | 9.3 | 9.1 | 9.0 | 10.0 | 10.6 | 9.4 | 8.4 |
| Total volume of alkaline material/polysaccharide derivative (mass ratio) | | 0.12 | 0.06 | 0.02 | 0.11 | 0.10 | 0.10 | 0.14 | 0.21 | 0.16 | 0.29 |
| Divalent anionic carbonate/polysaccharide derivative (mass ratio) | | 0.12 | 0.06 | 0.02 | 0.06 | 0.04 | 0.03 | 0.06 | 0.06 | 0 | 0 |
| Visual observability test | | O | O | X | O | O | O | O | O | X | X |
| Usable time | 0 minute | O | O | X | O | O | O | O | O | O | X |
| | 20 min | O | O | X | O | O | X | O | O | O | X |
| | 30 min | O | O | X | O | O | X | O | O | X | X |
| | 60 min | O | O | X | O | O | X | X | X | X | X |

## Claims

1. A material for medical treatment comprising a crosslinking composition comprising at least two components of a crosslinking polysaccharide derivative with an active ester group reactive with active hydrogen in the co-presence of an alkali, where the active ester group is preliminarily introduced in a side chain of a polysaccharide and the crosslinking polysaccharide derivative is not yet crosslinked, and a carbonate including at least one type of carbonate generating divalent carbonate ion in an amount at a mass ratio of 0.03 or more to the polysaccharide derivative.

2. A material for medical treatment according to claim 1, where the carbonate generating divalent carbonate ion is sodium carbonate.

3. A material for medical treatment according to claim 1 or 2, where the carbonate additionally includes a hydrogen carbonate.

4. A material for medical treatment according to claim 3, where the hydrogen carbonate is contained at a molar ratio of 0.5 to 1 to the carbonate generating divalent carbonate ion.

5. A material for medical treatment according to any of claims 1 to 4, additionally comprising an alkali salt other than the carbonate.

6. A material for medical treatment according to any of claims 1 to 5, where the crosslinking polysaccharide derivative further contains carboxyl group and/or carboxymethyl group.

7. A material for medical treatment according to any of claims 1 to 6, where the crosslinking polysaccharide derivative further contains carboxylate group in an amount of 0.1 to 5 mmol/g as the total amount of the active ester group-induced carboxylate group and no such active ester group-induced carboxylate group to the dry mass of the polysaccharide derivative.

8. A material for medical treatment according to any of claims 1 to 7, where the polysaccharide is dextrin.

9. A material for medical treatment according to any of claims 1 to 8, where the material for medical treatment is an adhesion preventive material and/or a hemostatic material and/or a clinically adhesive material.

10. A material for medical treatment according to any of claims 1 to 9, where the material for medical treatment is a fluid composition for spraying.

**11.** A visually observable crosslinked product formed from a material for medical treatment according to any of claims 1 to 10, where carbonate gas is encapsulated.

**Patentansprüche**

**1.** Material zur medizinischen Behandlung, das eine Vernetzungszusammensetzung umfasst, die wenigstens zwei Bestandteile eines vernetzenden Polysaccharidabkömmlings mit einer aktiven Estergruppe, die mit aktivem Wasserstoff bei der gleichzeitigen Anwesenheit eines Alkalis reaktionsfähig ist, wobei die aktive Estergruppe vorher in einer Seitenkette eines Polysaccharids eingeführt wird und der vernetzende Polysaccharidabkömmling noch nicht vernetzt ist, und ein Carbonat, das wenigstens eine Art von Carbonat erzeugendem zweiwertigem Carbonation in einer Menge bei einem Massenverhältnis von 0,03 oder mehr zu dem Polysaccharidabkömmling umfasst.

**2.** Material zur medizinischen Behandlung nach Anspruch 1, wobei das Carbonat erzeugende zweiwertige Carbonation Natriumcarbonat ist.

**3.** Material zur medizinischen Behandlung nach Anspruch 1 oder 2, wobei das Carbonat zusätzlich ein Hydrogencarbonat einschließt.

**4.** Material zur medizinischen Behandlung nach Anspruch 3, wobei das Hydrogencarbonat bei einem Molverhältnis von 0,5 bis 1 zu dem Carbonat erzeugenden zweiwertigen Carbonation enthalten ist.

**5.** Material zur medizinischen Behandlung nach einem der Ansprüche 1 bis 4, das zusätzlich ein anderes Alkalisalz als das Carbonat umfasst.

**6.** Material zur medizinischen Behandlung nach einem der Ansprüche 1 bis 5, wobei der vernetzende Polysaccharidabkömmling ferner eine Carboxylgruppe und/oder Carboxymethylgruppe enthält.

**7.** Material zur medizinischen Behandlung nach einem der Ansprüche 1 bis 6, wobei der vernetzende Polysaccharidabkömmling ferner eine Carboxylatgruppe in einer Menge von 0,1 bis 5 mmol/g als die Gesamtmenge der durch die aktive Estergruppe induzierten Carboxylatgruppe und keiner solchen durch die aktive Estergruppe induzierten Carboxylatgruppe zu der Trockenmasse des vernetzenden Polysaccharidabkömmlings enthält.

**8.** Material zur medizinischen Behandlung nach einem der Ansprüche 1 bis 7, wobei das Polysaccharid Dextrin ist.

**9.** Material zur medizinischen Behandlung nach einem der Ansprüche 1 bis 8, wobei das Material zur medizinischen Behandlung ein Haftung verhinderndes Material und/oder ein hämostatisches Material und/oder ein klinisch haftendes Material ist.

**10.** Material zur medizinischen Behandlung nach einem der Ansprüche 1 bis 9, wobei das Material zur medizinischen Behandlung eine Fluidzusammensetzung zum Sprühen ist.

**11.** Visuell zu beobachtendes vernetztes Erzeugnis, das aus einem Material zur medizinischen Behandlung nach einem der Ansprüche 1 bis 10 geformt ist, wobei Carbonatgas eingekapselt ist.

**Revendications**

**1.** Matériau de traitement médical comprenant une composition de réticulation comportant au moins deux composants d'un dérivé de polysaccharide réticulable ayant un groupe ester actif capable de réagir avec un hydrogène actif en coprésence d'un alcali, où le groupe ester actif est préalablement introduit dans une chaîne latérale d'un polysaccharide et le dérivé de polysaccharide réticulable n'est pas encore réticulé, et un carbonate comprenant au moins un type de carbonate produisant un ion carbonate divalent en une quantité à un rapport de masse de 0,03 ou plus par rapport au dérivé de polysaccharide.

**2.** Matériau de traitement médical selon la revendication 1, dans lequel le carbonate produisant un ion carbonate divalent est un carbonate de sodium.

**3.** Matériau de traitement médical selon la revendication 1 ou 2, dans lequel le carbonate comprend en outre un carbonate d'hydrogène.

**4.** Matériau de traitement médical selon la revendication 3, dans lequel le carbonate d'hydrogène est contenu à un rapport molaire de 0,5 à 1 par rapport au carbonate produisant un ion carbonate divalent.

**5.** Matériau de traitement médical selon l'une quelconque des revendications 1 à 4, comportant en outre un sel alcali autre que le carbonate.

**6.** Matériau de traitement médical selon l'une quelconque des revendications 1 à 5, dans lequel le dérivé de polysaccharide réticulable comporte en outre un groupe carboxyle et/ou un groupe carboxyméthyle.

**7.** Matériau de traitement médical selon l'une quelconque des revendications 1 à 6, dans lequel le dérivé de polysaccharide réticulable contient en outre un groupe carboxylate en une quantité de 0,1 à 5 mmol/g en tant que quantité totale du groupe carboxylate induit par le groupe ester actif et aucun groupe carboxylate induit par le groupe ester actif par rapport à la masse sèche du dérivé de polysaccharide.

**8.** Matériau de traitement médical selon l'une quelconque des revendications 1 à 7, dans lequel le polysaccharide est la dextrine.

**9.** Matériau de traitement médical selon l'une quelconque des revendications 1 à 8, où le matériau de traitement médical est un matériau de prévention d'adhérence et/ou un matériau hémostatique et/ou un matériau cliniquement adhésif.

**10.** Matériau de traitement médical selon l'une quelconque des revendications 1 à 9, où le matériau de traitement médical est une composition fluide à pulvériser.

**11.** Produit réticulé visuellement observable formé à partir d'un matériau de traitement médical selon l'une quelconque des revendications 1 à 10, où le gaz carbonate est encapsulé.

FIG. 1

(A)

(B)

**EP 2 269 665 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005087289 A
- US 2003012734 A
- US 2006178339 A
- US 2008058469 A

**Non-patent literature cited in the description**

- *Biochemistry,* 1975, vol. 14 (7), 1535-1541